Europäisches Patentamt

(19) European Patent Office  (11) Publication number: **0 118 258**

Office européen des brevets  **B1**

(12)  # EUROPEAN PATENT SPECIFICATION

(45) Date of publication of patent specification: **08.04.87**  (51) Int. Cl.⁴: **C 07 C 29/15,** C 07 C 31/20,
C 07 C 69/14, C 07 C 69/16,
(21) Application number: **84301166.9**  C 07 C 67/00

(22) Date of filing: **23.02.84**

(54) Catalytic process for the preparation of ethylene glycol.

(30) Priority: **03.03.83 GB 8305874**

(43) Date of publication of application:
**12.09.84 Bulletin 84/37**

(45) Publication of the grant of the patent:
**08.04.87 Bulletin 87/15**

(84) Designated Contracting States:
**DE FR GB IT NL**

(56) References cited:
**EP-A-0 012 924**
**EP-A-0 033 425**
**EP-A-0 053 386**

(73) Proprietor: **IMPERIAL CHEMICAL INDUSTRIES PLC**
**Imperial Chemical House Millbank**
**London SW1P 3JF (GB)**

(72) Inventor: **Whyman, Robin**
**23 Rowan Park Quarry Lane**
**Christleton NR. Chester (GB)**

(74) Representative: **Stephenson, Kenneth et al**
**Imperial Chemical Industries PLC Legal**
**Department: Patents PO Box 6 Bessemer Road**
**Welwyn Garden City Herts, AL7 1HD (GB)**

Courier Press, Leamington Spa, England.

**Description**

This invention relates to a catalytic process and more particularly to a catalytic process for the selective production of ethylene glycol from synthesis gas.

The catalytic conversion of synthesis gas (a mixture of carbon monoxide and hydrogen) to useful organic materials is well known. In particular, methanol has been commercially manufactured from synthesis gas by a heterogeneous catalytic reaction for many years. More recently, motivated largely by the increase in the price of oil, processes have been proposed with the object of producing not methanol but organic compounds having two or more carbon atoms in the molecule. All of the processes that have been proposed produced a mixture of products, the constitution of this mixture varying considerably depending on the reaction conditions and the specific catalyst employed. In the majority of these processes, the catalyst contains a metal of Group VIII of the Periodic Table and the products obtained have included methanol, ethanol, propanol, higher alcohols (up to at least $C_{27}$ products), ethylene glycol, propylene glycol, glycerol, ethers and esters of these alcohols, acetaldehyde and acetic acid. Several processes have had the specific object of increasing the proportion of ethylene glycol in the reaction product at the expense of less valuable materials.

Many of the processes aimed at improving glycol selectivity make use of a catalyst based on rhodium. Thus, United States Patent No. 3 833 634 describes a process for preparing glycols by reacting carbon monoxide with hydrogen in the presence of a rhodium carbonyl complex catalyst. Rhodium, however, is an expensive metal and considerable effort has been directed towards the partial or complete replacement of rhodium by other Group VIII metals.

European Patent Application 33425A describes a process for the selective production of ethylene glycol and/or esters thereof by contacting carbon monoxide and hydrogen with a catalyst comprising a mixture of Group VIII metals of which ruthenium is the major component, the preferred minor component being rhodium. As in other processes of this type, the catalyst is usually used in conjunction with a co-catalyst which is a compound of one or more of the metals of Group IA, IIA or IIB of the Periodic Table or a nitrogen-containing cation and/or base. Metal compounds mentioned include oxides, hydroxides, carbonates, bicarbonates and acetates with a preference for carboxylates when carboxylic acids are employed as reaction media. Compounds based on nitrogen-containing cations and/or bases include salts wherein the anion is hydroxide, nitrate, halide or carboxylate. The co-catalysts used in the Examples are metal carboxylates (acetates, carbonates or benzoates) or pyridine type compounds, the reaction medium usually being acetic acid. There is no illustration of the use of a nitrate and no indication of what particular effect may be attributed to such a compound.

It has now been found that when rhodium is used as the principal catalytic species, the ethylene glycol selectivity can be significantly improved by the inclusion of nitrate ions.

Thus, according to the invention, there is provided a method for the preparation of ethylene glycol and/or a carboxylic acid ester thereof which comprises introducing carbon monoxide, hydrogen, a liquid medium comprising a carboxylic acid, a catalyst containing rhodium as the major metallic component and nitrate ions into a reaction zone and subjecting said reaction zone to temperature in the range 100—400°C and a pressure of at least 50 bars.

The molar ratio of carbon monoxide to hydrogen may suitably be in the range 1:5 to 5:1 although ratios outside this range can be used. The reaction may also be carried out in the presence of gaseous inert diluents, for example carbon dioxide.

The carboxylic acid liquid reaction medium may suitably be an aliphatic monocarboxylic acid, having 1—12 carbon atoms for example acetic acid but other carboxylic acids, for example substituted aliphatic monocarboxylic acids, dicarboxylic acids and aromatic or heterocyclic carboxylic acids, may be used if desired. Other liquids may be used together with the carboxylic acid, for example other solvents known in synthesis gas chemistry, for example water, ketones, ethers, lactones or amides.

The rhodium, may be used in elemental form or in the form of a compound, for example a salt or complex, thereof. Suitable salts and complexes are known in the art and include rhodium acetate, tetrarhodium dodecarbonyl and rhodium tris (acetylacetonate). The amount of rhodium used is not critical and may vary over a wide range. In general, the amount used is the minimum that will provide an acceptable reaction rate. Minor amounts of other metals or compounds therof, known as catalysts in synthesis gas chemistry, may be used in conjunction with the rhodium. Suitable additives include other Group VIII metals and their compounds. The effect of the nitrate ion is most marked, however, when rhodium or a compound thereof constitutes substantially the whole of the catalytic entity.

Nitrate ions may be introduced into the reaction zone in the form of nitric acid or a salt thereof. Suitable salts include nitrates of alkali metals, alkaline earth metals, Group VII metals, Group VIII metals, for example rhodium, rare earth metals and organic cations. The effect of the nitrate ion compared with other anions is particularly marked in the case of the free acid and Group IIA, Group IIB and rare earth metal salts such as the lanthanum and cerium salts. The molar ratio of nitrate to rhodium is not critical and is suitably within the range 0.1:1 to 100:1, especially 0.5:1 to 30:1.

Preferred reaction temperatures are in the range 150—300°C. Reaction pressures are preferably less than 3000 bars, the preferred range being 300—1200 bars.

The process may be operated in a continuous or batchwise manner. It may also be carried out in either

2

a homogeneous or a heterogeneous reaction system, the former being preferred. Accordingly, the catalyst may be present in the system as a heterogeneous phase for example as a metal or compound deposited on a solid support such as carbon, silica or alumina or may be dissolved in the liquid medium and thus form a homogeneous phase therewith. If a heterogeneous catalyst is used, the components of the reaction mixture can be separated from each other and from the catalyst by conventional means, for example separating the catalyst by filtration and fractionally distilling the organic components in the mixture. When a homogeneous system is used, the catalyst will remain in the residue after volatile products have been distilled out, collected and separated. The catalyst may be re-used by regeneration of suitable compounds and with fresh addition of the liquid medium.

Conveniently, the catalyst and nitrate may be immersed in the medium in an autoclave pressurised to the required high pressure and the mixture of reactant gases carbon monoxide and hydrogen passes into the liquid medium. The temperature may be raised as required and maintained for the period of the reaction after which both temperature and pressure are lowered and the product mixture removed from the autoclave. The organic products may be separated by distillation and the catalyst recovered for re-use by conventional means.

The effect of the nitrate ion is to improve the ethylene glycol selectivity to a significant extent, the effect being much greater than is the case when nitrates are included in ruthenium catalysed systems. Any glycol obtained as carboxylic acid esters may be converted to glycol itself by standard hydrolytic procedures.

The invention is illustrated but not limited by the following Examples:

Examples 1—10

A typical preparation was conducted as follows:

Rhodium dicarbonyl acetylacetonate (0.052 g; 0.2mmole), lanthanum nitrate hexahydrate (0.087 g; 0.2mmole) and glacial acetic acid (52.5 g) were charged into a 100 ml capacity silver lined stainless steel autoclave fitted with a flip-flop stirrer. The autoclave was sealed and after being purged four times with a CO/H$_2$ mixture was pressurised to about 500 bars with synthesis gas in a 1:1 ratio of CO to H$_2$. The autoclave was then heated to 230°C and the pressure was increased to 1000 bars by further addition of synthesis gas. Pressure and temperature were maintained for 4 hours after which the autoclave was allowed to cool to ambient temperature. The autoclave was carefully vented, the product discharged and analysed by gas chromatography using a 15% Carbowax 20M on Chromosorb W HP column.

Other formulations were examined using the same procedure, the results being summarised in the following Table. In the above description, the figures in parantheses refer to Example 4 in the Table. Examples 1—3, 6, 11, 13, 15, and 17 are not illustrative of the invention but are included for the purpose of comparison. In Example 10, the rhodium dicarbonyl acetylacetonate was replaced by rhodium nitrate dihydrate, no additional co-catalyst being used. The figures in the Table clearly show the improved glycol selectivity given by formulations containing nitrate ions compared with Examples 1—3, 6, 11, 13, 15 and 17, which contain no nitrate ion.

3

| Example | Rh | Promoter (mmole) | MeOAc | EtOAc (mole/1/hr) | $(CH_2OAc)_2$ | $CH_2OAc$–$CH_2CH$ | Selectivity EG/MeOH |
|---|---|---|---|---|---|---|---|
| 1 | 0.2 | — | 0.246 | 0.009 | 0.048 | 0.005 | 0.22 |
| 2 | 0.2 | 0.2 $La(OAc)_3$ | 0.245 | 0.010 | 0.043 | 0 | 0.18 |
| 3 | 0.2 | 2.0 $La(OAc)_3$ | 0.315 | 0.013 | 0.070 | 0 | 0.22 |
| 4 | 0.2 | 0.2 $La(NO_3)_3 6H_2O$ | 0.169 | 0.013 | 0.114 | 0.006 | 0.71 |
| 5 | 0.2 | 2.0 $La(NO_3)_3\ 6H_2O$ | 0.119 | 0.023 | 0.123 | 0.011 | 1.13 |
| 6 | 0.2 | 2.0 $Ce(OAc)_3 1.5H_2O$ | 0.175 | 0.010 | 0.045 | 0.004 | 0.28 |
| 7 | 0.2 | 2.0 $Ce(NO_3)_3 6H_2O$ | 0.086 | 0.025 | 0.094 | 0.004 | 1.14 |
| 8 | 0.2 | 2.0 $HNO_3$ | 0.118 | 0.015 | 0.104 | 0.007 | 0.94 |
| 9 | 0.2 | 6.0 $HNO_3$ | 0.077 | 0.023 | 0.088 | 0.006 | 1.19 |
| 10 | 0.2 | $Rh(NO_3)_3 2H_2O$ | 0.150 | 0.012 | 0.080 | 0.015 | 0.63 |
| 11 | 0.2 | 2.0 $NaOAc.3H_2O$ | 0.032 | 0.009 | 0.041 | 0.0 | 1.28 |
| 12 | 0.2 | 2.0 $NaNO_3$ | 0.025 | 0.012 | 0.039 | 0.003 | 1.69 |
| 13 | 0.2 | 2.0 $Mg(OAc)_2.4H_2O$ | 0.106 | 0.013 | 0.092 | 0.007 | 0.83 |
| 14 | 0.2 | 2.0 $Mg(NO_3)_2$ | 0.059 | 0.018 | 0.076 | 0.033 | 1.86 |
| 15 | 0.2 | 2.0 $Zn(OAc)_2.2H_2O$ | 0.117 | 0.016 | 0.066 | 0.015 | 0.69 |
| 16 | 0.2 | 2.0 $Zn(NO_3)_2.6H_2O$ | 0.061 | 0.019 | 0.073 | 0.034 | 1.76 |
| 17 | 0.2 | 2.0 $Mn(OAc)_2$ | 0.111 | 0.005 | 0.031 | 0.011 | 0.038 |
| 28 | 0.2 | 2.0 $Mn(NO_3)_2.4H_2O$ | 0.055 | 0.019 | 0.069 | 0.008 | 1.41 |

## Comparative Examples

The following Table shows that when ruthenium was used as the sole catalytic entity (ruthenium trisacetylacetonate), the nitrate ion did not improve glycol selectivity.

| Ru | Promoter (mmole) | MeOAc | EtOAc | $(CH_2OAc)_2$ | $\dfrac{CH_2OAc}{CH_2OH}$ | Selectivity EG/MeOH |
|---|---|---|---|---|---|---|
| | | | | mole/1/hr | | |
| 2.0 | | 0.180 | 0.008 | 0.015 | 0.002 | 0.09 |
| 2.0 | 2.0 $La(OAc)_3$ | 0.203 | 0.006 | 0.021 | 0.002 | 0.11 |
| 2.0 | 2.0 $La(NO_3)_3.6H_2O$ | 0.190 | 0.024 | 0.014 | 0.002 | 0.008 |

**Claims**

1. A method for the preparation of ethylene glycol and/or a carboxylic acid ester thereof which comprises introducing carbon monoxide, hydrogen, a liquid medium comprising a carboxylic acid, a catalyst containing rhodium as the major metallic component and nitrate ions into a reaction zone and subjecting said reaction zone to a temperature in the range 100—400°C and a pressure of at least 50 bars.

2. A method according to claim 1 wherein the nitrate ion is provided in the form of nitric acid or a Group IIa, Group IIB or rare earth metal salt.

4

**0 118 258**

**Patentansprüche**

1. Verfahren zur Herstellung von Ethylenglykol und/oder eines Carbonsäureesters davon, bei dem Kohlenmonoxid, Wasserstoff, ein flüssiges Medium, das eine Carbonsäure enthält, ein Katalysator, der als metallischen Hauptbestandteil Rhodium enthält, und Nitrationen in eine Reaktionszone eingeführt werden und die erwähnte Reaktionszone einer Temperatur im Bereich von 100 bis 400°C und einem Druck von mindestens 50 bar ausgesetzt wird.

2. Verfahren nach Anspruch 1, bei dem das Nitration in Form von Salpetersäure oder eines Salzes eines Metalls der Gruppe IIA oder der Gruppe IIB oder eines Seltenerdmetalls bereitgestellt wird.

**Revendications**

1. Procédé de préparation d'éthylèneglycol et/ou d'un ester d'acide carboxylique d'éthylèneglycol, qui consiste à introduire de l'oxyde de carbone, de l'hydrogène, un milieu liquide comprenant un acide carboxylique, un catalyseur contenant du rhodium comme principal composant métallique et des ions nitrate dans une zone de réaction, et à soumettre ladite zone de réaction à une température comprise dans l'intervalle de 100 à 400°C et à une pression d'au moins 50 bars.

2. Procédé suivant la revendication 1, dans lequel l'ion nitrate est introduit sous la forme d'acide nitrique ou d'un sel d'un métal du Groupe IIa, d'un métal du Groupe IIb ou d'un métal du groupe des terres rares.